# FASCICULE DE BREVET EUROPEEN

(11) **EP 3 487 313 B1**
(45) Date de publication et mention de la délivrance du brevet: **11.10.2023**
(21) Numéro de dépôt: 17706553.9
(22) Date de dépôt: 20.01.2017
(51) Int. Cl.: A23K 40/20, A23K 40/30, A23K 50/40, A61K 9/00

(54) **SUPPORT POUR L'ABSORPTION ORALE D'UNE SUBSTANCE ACTIVE PAR DES ANIMAUX, SON PROCÉDÉ DE PRÉPARATION ET SES UTILISATIONS**
TRÄGER ZUR ORALEN VERABREICHUNG EINES WIRKSTOFFS AN EIN TIER, VERFAHREN ZUR DESSEN HERSTELLUNG, UND DESSEN VERWENDUNGEN
DELIVERY VEHICLE FOR THE ORAL ADMINISTRATION OF AN ACTIVE TO AN ANIMAL, PROCESS FOR ITS PREPARATION AND ITS USES

(30) Priorité: 19.07.2016 FR 1656867
(43) Date de publication de la demande: 29.05.2019
(73) Titulaire: Galewpet, 22600 Loudeac (FR)
(72) Inventeur: GABILLET, Hervé, 56120 Lanouee (FR)
(74) Mandataire: Cabinet Nony
(86) Numéro de dépôt international: PCT/FR2017/050121
(87) Numéro de publication internationale: WO 2018/015620

(56) Documents cités:
- EP-A1- 0 796 565
- EP-A2- 0 320 320
- WO-A1-2016/020217
- US-A- 4 362 748
- US-A1- 2003 129 295
- US-A1- 2008 044 481
- TP LABUZA ET AL: "Moisture migration and control in multi-domain foods", TRENDS IN FOOD SCIENCE & TECHNOLOGY, vol. 9, no. 2, 1 février 1998 (1998-02-01) , pages 47-55, XP055313651, DOI: S0924-2244(98)00005-3

## Description

### Domaine technique

La présente invention se rapporte à un support permettant de faciliter l'absorption orale d'au moins une substance active par des animaux, ainsi qu'à son procédé de préparation et à ses utilisations.

La présente invention trouve des applications dans le domaine vétérinaire, notamment dans le domaine de la nutrition animale et de la médecine vétérinaire.

### Etat de la technique

Le nombre des animaux domestiques tels que chiens et chats est en constante augmentation. Parallèlement à cette augmentation, on assiste à une évolution des soins apportés à ces animaux.

Toutefois, le suivi des traitements administrés n'est pas toujours bien assuré en raison de la difficulté à faire absorber les substances médicamenteuses à l'animal. Il est en effet bien connu que nombre de ces substances exercent un effet répulsif pour l'odorat des chiens ou des chats qui refusent de les absorber, rendant ainsi très difficile, voire impossible, le respect d'un traitement.

Il existe donc un besoin pour un conditionnement des substances actives apte à déjouer la vigilance des animaux, en particulier des chiens, des chats, des chevaux, des porcs et des ruminants.

Le document EP 0 320 320 décrit un comprimé comportant un noyau, contenant les substances actives, et une matrice appétente enrobant complètement ce noyau. Toutefois, ce produit est difficile à fabriquer car il est peu envisageable d'enrober extemporanément et à l'unité des médicaments livrés dépourvus de matrice appétente.

Le document EP 0 574 301 décrit par ailleurs une pastille constituée d'une matrice en un matériau appétent comportant un logement de forme sensiblement complémentaire à celle du comprimé à faire avaler à l'animal. Le comprimé est bloqué en force dans le logement puis la pastille est présentée à l'animal de manière à cacher le comprimé. Une telle pastille présente plusieurs inconvénients. En premier lieu, la pastille est fabriquée avec son logement. Il n'est plus possible ensuite de modifier la forme de ce dernier, en particulier pour l'adapter à des comprimés de formes différentes. En second lieu, si l'animal mord dans la pastille, il peut la rompre et provoquer le détachement de la substance active. Il peut alors sentir cette substance active et la recracher.

Le document WO2016/020217 décrit des formes d'administration d'actifs à des chats, chiens ou humains, dont la composition rend le produit plus acceptable. Ces produits comportent un noyau comprenant un biopolymère et un actif lipophile. L'enveloppe est une matrice comprenant un biopolymère.

Le document Labuza et al. : « Moisture migration and control in multi-domain foods », Trends in food science & technology, vol. 9, no. 2, enseigne que le contrôle de l'humidité initiale et de la migration de l'humidité est un paramètre critique pour la qualité et la sécurité de la nourriture. Les deux facteurs principaux influençant la quantité et le taux de la migration de l'humidité sont l'équilibre de l'activité de l'eau (thermodynamique) et les facteurs affectant le taux de diffusion (dynamiques de transfert de masse).

Il existe donc un réel besoin d'un produit palliant ces défauts, inconvénients et obstacles de l'art antérieur, en particulier permettant de faciliter l'absorption orale d'au moins une substance par des animaux.

### Description de l'invention

Aux termes d'importantes recherches, la Demanderesse est parvenu à mettre au point un support permettant de remédier aux inconvénients.

Le support de l'invention permet avantageusement de faciliter l'absorption orale d'au moins une substance par des animaux, et plus avantageusement de deux ou plus de deux substances actives simultanément par des animaux.

De manière surprenante, le support de l'invention permet notamment l'absorption simultanée de substances dont l'administration est généralement considérée comme incompatible, comme par exemple les huiles essentielles et les probiotiques.

De plus, le support de l'invention permet de manière avantageuse de conserver de manière stable d'un point de vue bactériologique une ou plusieurs substances actives en son sein, en particulier sans que les substances actives ne se mélangent.

Le support de l'invention est de manière particulièrement avantageuse absorbée spontanément par les animaux, et ce bien qu'il puisse renfermer des substances actives dont le goût, l'odeur ou la texture dans une formulation classique ne sont pas prisées par les animaux.

Ainsi, un premier objet de l'invention se rapporte à un support permettant de faciliter l'absorption orale d'au moins une substance active par des animaux, caractérisé en ce qu'il comprend :
- au moins une bille comprenant ladite au moins une substance active,
- une matrice appétente gélifiée entourant ladite au moins une bille, le pH de ladite matrice étant inférieur à 4,
dans lequel l'eau résiduelle (Aw) de ladite matrice et de ladite au moins une bille ont une valeur identique et comprise de 0,4 à 0,9.

Le support de l'invention a avantageusement une forme et une taille permettant son absorption orale par un animal. La forme et la taille peuvent être adaptées en fonction de l'animal, notamment de sa nature, de sa taille et/ou de son âge, par l'homme du métier au vu des connaissances générales de ce domaine. A titre d'exemple, la forme du support peut être celle d'un os, d'un cylindre, d'une pastille, d'un dôme, d'un croissant, d'un parallélépipède, d'un triangle, d'une croix, d'une sphère, d'un palet, d'un disque, d'une étoile, ou d'un cube, cette liste n'étant pas limitative. Le support peut être présenté comme une friandise pour les animaux, et de ce fait avoir une forme et une taille adaptée pour cela.

On entend par « animaux », au sens de la présente invention, les animaux à l'exclusion de l'être humain. Il peut s'agir des animaux domestiques ou d'élevage, comme par exemple le chien, le chat, la souris domestique, le rat domestique, le hamster, le cochon d'inde, la gerbille, le furet, le cheval, l'âne, la vache, le lama, le porc, le lapin, le canard, le pigeon, la perruche, le paon, les poissons et les crustacés. De manière préférée, il peut s'agir du chien ou du chat.

On entend par « substance active », au sens de la présente invention, toute substance active ayant un impact sur la santé et/ou la nutrition et/ou le bien-être de l'animal. Il peut s'agir par exemple d'une substance :
- permettant le traitement ou la prévention d'une maladie de l'animal, et/ou
- permettant le soin, par exemple pour l'hygiène buco-dentaire de l'animal, notamment pour éviter/diminuer le tartre et/ou améliorer l'haleine de l'animal, et/ou
- à visée cosmétique, notamment du pelage, par exemple pour améliorer la brillance du poil, et/ou pour rétablir l'hydratation de la peau en renforçant la fonction barrière cutanée, et/ou pour freiner la chute des poils (hors mue saisonnière) et soutenir la repousse, et/ou pour diminuer la présence de pellicules, et/ou pour désodoriser le poil en respectant l'odorat de l'animal, et/ou protéger le pelage par un effet antioxydant, et/ou
- à visée bien-être, par exemple ayant une action contre le stress, et/ou contre le surpoids de l'animal, ou pour améliorer la digestion de l'animal, et/ou pour améliorer le tonus de l'animal, et/ou
- ayant une action dans un cadre post-opératoire, par exemple suite à une endoscopie digestive, une endoscopie respiratoire, un scanner, une myélographie, un myéloscanner, une ponction de LCR, une arthroscopie, un électrorétinogramme, un bilan radiographique orthopédique, une radiographie de dépistage de la
dysplasie de hanche, ou encore par exemple suite à une chirurgie des tissus mous, par exemple un traitement de l'aspergillose nasale simple, une exérèse du conduit auditif et bullectomie, un traitement du syndrome des races brachycéphales, un traitement de la persistance du canal artériel, une péricardectomie sous thoracoscopie, une lobectomie pulmonaire, une biopsie foie, du pancréas, ou du rein sous coelioscopie, un shunt portosystémique, une chirurgie des voies biliaires, un traitement de hernie périnéale, une ovariectomie sous coelioscopie, une gastropexie de convenance sous coelioscopie, un traitement de l'uretère ectopique, une chirurgie de l'incontinence, une chirurgie prostatique (kyste, abcès, etc.), une chirurgie cutanée avec lambeau ou greffe de peau, et/ou une reconstruction de la paroi thoracique ou abdominale, ou encore par exemple suite à une chirurgie ophtalmologique, comme par exemple un traitement d'entropions, de cil ectopique, de papillome palpébral, d'une tumeur et greffe palpébrale, d'une luxation de la glande nictitante, d'une malposition de la membrane nictitante, d'une dérivation du canal de Sténon, d'une plaie cornéenne, d'un volet de greffe conjonctivale, d'une kératectomie lamellaire, d'une greffe de cornée, d'une chirurgie de la cataracte, comme une phakoémulsification simple, une luxation du cristallin, une prothèse endoculaire, une chirurgie du strabisme secondaire à une luxation du globe, ou encore par exemple suite à une chirurgie orthopédique, par exemple suite à une fracture, une rupture du ligament croisé, une luxation de rotule, une luxation de hanche, une ostéochondrite disséquante, une ostéotomie pelvienne, une prothèse totale de hanche, ou encore par exemple une chirurgie neuro-rachidienne comme une hémilaminectomie, une corpectomie, ou une luxation/fracture vertébrale, ou encore par exemple suite à un traitement d'une pathologie rénale, comme un MBAUF (Maladies du Bas Appareil Urinaire Félin), une urolithiase, une IRA ( insuffisance renal aiguë) ou une cystite idiopathique.

La substance peut être notamment choisie dans le groupe comprenant les probiotiques, les huiles essentielles, les médicaments, les nutraceutiques et les nutriments. Le support de l'invention peut contenir soit une seule de ces substances actives, soit plusieurs de ces substances actives, par exemple 2 ou 3 ou 4 ou 5.

Parmi les probiotiques, il peut s'agir de tout probiotiques utilisables chez les animaux. Il peut s'agir par exemple de Bifidobactéries, tels que L. acidophilus et L. bulgaricus, L. casei et Bifidobacterium bifidus, Enterococcus faecium, Saccharomyces cerevisiae ou Bacillus subtilis.

Parmi les huiles essentielles, il peut s'agir toute huile essentielle utilisable chez les animaux. Il peut s'agir par exemple de l'huile essentielle d'Ahibéro, de l'huile essentielle de basilic tropical, de l'huile essentielle de Camomille romaine, de l'huile essentielle de Cannelle de Chine, de l'huile essentielle de Cèdre de l'Atlas, de l'huile essentielle de Ciste ladanifère, de l'huile essentielle de Citron (zestes), de l'huile essentielle de Citronnelle de Ceylan, de l'huile essentielle d'Eucalyptus citronné, de l'huile essentielle d'Eucalyptus mentholé, de l'huile essentielle de Gaulthérie couchée, de l'huile essentielle de Genévrier, de l'huile essentielle de Géranium rosat, de l'huile essentielle de Girofle clous, de l'huile essentielle d'Hysope couchée, de l'huile essentielle d'lary, de l'huile essentielle d'immortelle (Hélichryse italienne), de l'huile essentielle de Katafray, de l'huile essentielle de Laurier noble, de l'huile essentielle de Lavande aspic, de l'huile essentielle de Lavande vraie, de l'huile essentielle de Lavandin super, de l'huile essentielle de Lemongrass, de l'huile essentielle de Menthe poivrée, de l'huile essentielle de Niaouli, de l'huile essentielle d'Origan compact, de l'huile essentielle de Palmarosa, de l'huile essentielle de Ravintsara, de l'huile essentielle de Tanaisie annuelle, de l'huile essentielle de Tea tree ou de l'huile essentielle de Thym à linalol.

Parmi les médicaments, il peut s'agir de tout médicament utilisable chez les animaux. Il peut s'agir par exemple d'agents anti-infectieux, comme les antibiotiques ou les sulfamides, d'agents anti-parasitaires internes et/ou externes, d'agents anti-inflammatoires ou anti-histaminiques, de vaccins oraux, d'hormones, de substances de thérapie digestive, comme les pansements et sédatifs gastro intestinaux, les flores de remplacement, les antidiarrhéiques, les hépato-protecteurs, les antispasmodiques, les laxatifs, les antiseptiques intestinaux et les réhydratants oraux, les substances de thérapie rénale, les substances de thérapie cardio-vasculaire comme les analeptiques cardiaques et cardiovasculaires, les hémostatiques, les vasoconstricteurs et dilatateurs, les substances de thérapie respiratoire comme les analeptiques respiratoires, les antitussifs, les bronchodilatateurs, les bronchosécrétolytiques et les antiseptiques respiratoires, les substances agissant sur le système nerveux comme les analgésiques, les sédatifs et tranquillisants, les anti-épileptiques, les anesthésiques, orexigènes et anorexigènes, les substances de thérapie immunitaire comme les immunodépresseurs, les immunostimulants et les immunoglobulines de remplacement, les diurétiques, les substances anticancéreuses comme les antimitotiques et les substances de traitement des articuations, notamment contre l'arthrose comme la glucosamine, la chondroitine, ou les anti-inflammatoires.

Parmi les nutriments, il peut s'agir de tout nutriment utilisable chez les animaux. Il peut s'agir par exemple d'un ou plusieurs nutriments choisis parmi :
- les vitamines,
- les minéraux, comme le calcium, le phosphore ou le magnésium,
- les oligoéléments, comme le fer, le zinc, l'iode, le sélénium, le cuivre, le chrome, le manganèse, le cobalt ou le fluor,
- les protéines, d'origine animale comme les protéines d'oeuf, de viandes, de poissons ou du lait, ou d'origine végétale, comme les isolats de soja. Il peut s'agir par exemple d'acides aminés, comme l'arginine, la glutamine, la tyrosine, la phénylalanine, de carnitine, de taurine
- les lipides, comme les acides gras, par exemple les oméga 3 ou les oméga 6,
- les glucides, comme l'amidon, les fibres insolubles comme la cellulose brute ou la lignine, les fibres solubles, comme les fructooligosaccharides (FOS) ou les mannan-oligosaccharides (MOS), les sucres ou glucides simples,
- les parties ou extraits de plantes, par exemple l'Aloe vera, Valeriana officinalis, Passiflora incarnata, Rosmarinus sp., Vitis sp., Curcuma sp, Eugenia sp.part ou Citrus sp, cette liste n'étant pas limitative,
- les parties ou extraits d'algues, comme par exemple la spiruline.

Parmi les nutraceutiques, il peut s'agir de tout nutraceutique utilisable chez les animaux. Il peut s'agir par exemple d'un ou plusieurs nutraceutiques choisis parmi : le chitosan, la glucosamine, les chitooligosaccharides et le soja fermenté.

On entend par « bille », au sens de la présente invention, tout contenant renfermant une substance active, et de forme sensiblement sphérique. La bille peut avoir un diamètre compris d'environ 200 microns à environ 6 mm. La bille peut être un contenant plein ou un contenant creux. S'il s'agit d'un contenant creux, il peut s'agir par exemple d'une capsule, notamment une microcapsule, ou d'une vésicule. Avantageusement, il peut s'agir d'une microcapsule renfermant une substance active, dont la forme est définie par une enveloppe. S'il s'agit d'une contenant plein, la bille peut par exemple être enrobée, notamment pour maintenir la forme des billes et que le contenu des billes ne se disperse pas dans la matrice. Les moyens d'enrobage des billes peuvent être tout moyen permettant d'obtenir cet effet, comme par exemple les hydrocolloides comme l'alginate, la pectine, et/ou la cire végétale fondue.

La teneur en eau résiduelle (Aw) d'une bille est comprise de 0,4 à 0,9, par exemple de 0,5 à 0,8, ou de 0,55 à 0,75, ou de 0,6 à 0,75. Avantageusement, la valeur de 0,9 est exclue de la gamme. Avantageusement, cette gamme d'Aw permet une stabilité microbiologique optimale de la substance active contenue dans la bille. L'homme du métier peut choisir l'Aw parmi ces gammes de valeurs en fonction de la substance active contenue dans la bille, au moyen de ses connaissances générales du domaine technique. L'Aw peut être mesurée par l'homme du métier par tout procédé connu, comme par exemple au moyen d'un Aw mètre du type Labswift-aw de Novasina. L'Aw peut être obtenue ou contrôlée par tout moyen connu de l'homme du métier, par exemple au moyen d'au moins un humectant choisi parmi le monopropylene glycol et/ou le xilytol.

L'enveloppe de la bille peut être toute enveloppe permettant de définir le contour de la bille. L'enveloppe peut notamment permettre d'isoler l'intérieur de la bille de l'extérieur de la bille, c'est-à-dire de limiter mécaniquement les échanges entre l'intérieur et l'extérieur de la bille. L'enveloppe permet, de manière avantageuse, la conservation de l'Aw des billes.

L'enveloppe de la bille peut être constituée de ou comprendre tout matériau permettant le ou les effet(s) technique(s) précité(s). La bille peut par exemple être une bille gélifiée, c'est-à-dire dont l'enveloppe comprend ou est constituée d'un gélifiant, par exemple d'alginate, comme l'alginate de sodium ou l'alginate de calcium, et/ou de cire végétale, par exemple l'huile de jojoba, la cire de carnauba, la cire de candelilla, la cire de cera bellina, la cire de riz ou la cire de soja. De préférence, la bille est une capsule ou une microcapsule d'alginate et/ou de cire végétale. La quantité de gélifiant dans la bille ou son enveloppe peut être comprise de 0,1 à 5,0%, par exemple de 0,3 à 4,5%, ou de 1,0 à 4,0%, ou de 1,5 à 3,5%.

L'enveloppe peut comprendre un ou plusieurs colorant(s). Ainsi, une bille peut apparaître colorée. Avantageusement, une bille peut être colorée en fonction de la substance active qu'elle contient, afin d'identifier la nature et/ou le nombre de substance(s) active(s) présente(s) par support. Avantageusement, une bille peut être colorée de manière à être visible dans la matrice, ou au contraire à n'y être pas visible. Dans le premier cas, la bille a une couleur différente de la couleur de la matrice. Dans le second cas, la bille a une couleur identique ou quasi-identique à celle de la matrice. Une bille peut avoir une couleur choisie parmi le bleu, le rouge, le vert, le jaune, le rose, l'orange, le violet, le marron, le noir, le caramel, le blanc et le gris. La matrice peut être transparente ou opaque, et/ou avoir une couleur choisie parmi le bleu, le rouge, le vert, le jaune, le rose, l'orange, le violet, le caramel, le marron, le noir, le blanc et le gris.

Avantageusement, le support de l'invention peut contenir une bille ou plus d'une bille, par exemple au moins 2 billes, par exemple au moins 3, ou au moins 4, ou au moins 5 billes, ou au moins 10 billes, ou au moins 15 billes, ou plus, leur nombre pouvant être fonction de leur taille par rapport à celle du support. Par exemple, le support peut comprendre de 2 à 15 billes, ou de 2 à 10 billes, ou de 3 à 10 billes, ou de 4 à 10 billes, notamment si le diamètre des billes est compris de 2 mm à 6 mm. Alternativement, si le diamètre des billes est compris de 200 µm à 2 mm, le nombre de billes peut être supérieur à 15, ou supérieur à 20, ou supérieur à 50.

Avantageusement, un support peut contenir soit une seule substance active, si le support ne comprend qu'une seule bille ou si toutes les billes du support contiennent toutes la même substance active, ou plus d'une substance active si le support contient plusieurs billes qui contiennent chacune une substance active différente de celle des autres billes. Ainsi, le support peut contenir une substance active, ou 2 substances actives, ou au moins 2 substances actives, par exemple au moins 3 substances actives, ou au moins 4 substance actives, ou au moins 5 substances actives, par exemple 10 substances active.

Chaque bille peut contenir une quantité de substance active adaptée aux besoins de l'animal auquel le support est administré. Par exemple, chaque bille peut contenir de 20 à 70% en poids d'une substance active par rapport au poids total de la bille, par exemple de 30 à 60%, ou de 40 à 50%.

Le support comprend ainsi une matrice dans laquelle sont réparties une ou plusieurs billes. La matrice est donc un matériau englobant une ou plusieurs billes telles que définies ci-avant.

Avantageusement, la matrice peut en outre comprendre un matériau permettant la prévention et/ou la diminution du tartre sur la dentition de l'animal, par son effet abrasif potentiellement lié aux caractéristiques mécaniques du matériau. Il peut s'agir par exemple de cellulose micocristalline, comme le E 460, par exemple inclue sous forme de ces billes dans la matrice. La matrice est une matrice appétente, dont la texture, la consistance, le goût et l'odeur sont aptes à rendre le support attirant pour un animal et amène celui-ci à le manger facilement.

Avantageusement, le goût et l'odeur de la matrice peuvent être appétents du fait de la présence, dans la matrice, de facteurs d'appétence. Il peut s'agir par exemple de facteurs d'appétence à base de viande ou poisson ou crustacées, ou de facteurs d'appétence mimant le goût de viande, poisson, crustacées, pâté ou croquettes.

Avantageusement, la texture et la consistance de la matrice peuvent être appétentes du fait de son caractère agréable en bouche et agréable à mâcher pour l'animal. La matrice peut avoir par exemple une texture de type gel souple, comme du pudding, ou du flan, ou des bonbons gélifiés Gummy Bears^{®}.

La matrice peut être une matrice gélifiée, comprenant un gélifiant adapté, par exemple choisi parmi les carraghénanes, l'agar agar, la gomme gellane, la gélatine de boeuf, de porc ou de poisson, la gomme xanthane, la farine de graine de caroube, la gomme de guar, la gomme arabique, les alginates, la gomme adragante (également appelée tragacanthe) et les amidons. La quantité de gélifiant dans la matrice peut être comprise de 0,5 à 20%, par exemple de 1,0 à 18,0%, ou de 5,0 à 15,0%, ou de 7,0 à 13,0%.

Le pH de la matrice est inférieur ou égale à 4,0, ou alternativement strictement inférieur à 4,0, par exemple compris entre 0 et 3,8 ou entre 0,5 et 3,5, ou entre 1,0 et 3,0. Ce pH permet avantageusement au support d'être très stable dans le temps, par exemple au moins 2 ans, notamment d'un point de vue microbiologique. Le pH peut être mesuré par toute méthode connue de l'homme du métier, par exemple au moyen d'un pHmètre. Le pH peut être obtenu ou contrôlé par tout moyen connu de l'homme du métier, par exemple au moyen d'au moins un acide organique, comme par exemple l'acide propionique, l'acide isovalérique ou l'acide butyrique.

L'eau résiduelle (Aw) de la matrice a une valeur comprise de 0,4 à 0,9 par exemple de 0,5 à 0,8, ou de 0,55 à 0,75, ou de 0,6 à 0,75. La valeur de l'Aw est identique à celle des billes. Cette identité entre l'Aw de la matrice et l'Aw des billes permet de manière avantageuse d'éviter les échanges entre le contenu des billes et la matrice. En d'autres termes, chaque substance active reste dans la bille qui la contient et ne peuvent transférer dans la matrice. Ainsi, aucune substance active ne peut se retrouver en présence d'une ou plusieurs autres substance actives dans la matrice, ni se mélanger dans la matrice. L'Aw peut être mesurée par l'homme du métier par tout procédé connu, comme par exemple le AW mètre Labswift-aw de Novasina. L'Aw peut être obtenue ou contrôlée par tout moyen connu de l'homme du métier, par exemple au moyen d'au moins un humectant choisi parmi le monopropylene glycol et le xilytol.

Le support de l'invention permet ainsi de contenir, et donc d'administrer, en une seule prise, à un animal, plusieurs substances actives. Avantageusement, le support de l'invention peut comprendre des substances actives qui, en règle générale, ne sont pas compatibles pour une administration simultanée. Le support peut par exemple contenir un probiotique et une huile essentielle, chacune de ces substances se trouvant dans une bille et étant donc physiquement séparée de l'autre et dans l'impossibilité de se mélanger.

L'utilisation d'un support tel que défini précédemment, pour faciliter l'absorption orale d'au moins une substance active par des animaux, ladite substance active étant choisie parmi les probiotiques, les huiles essentielles, les nutraceutiques et les nutriments, **est décrite.** Un tel objet est une utilisation non thérapeutique. Avantageusement, la substance active peut être au moins un nutriment. Le support est administré à des animaux sains, c'est-à-dire non atteints par une pathologie, ou des animaux pour lesquels l'administration du support n'est pas réalisée dans le cadre d'un traitement thérapeutique ou prophylactique d'une pathologie.

Un autre objet de l'invention se rapporte à un support tel que défini précédemment, pour **son utilisation comme** médicament destiné aux animaux, **dans lequel ladite au moins une substance active comprise dans l'au moins une bille est au moins un médicament.** Dans ce mode de réalisation, le support est administré à des animaux sains ou atteints d'une pathologie, et l'administration du support est réalisée dans le cadre d'un traitement thérapeutique ou prophylactique d'une pathologie chez ces animaux.

Un autre objet de l'invention se rapporte à un procédé de préparation d'un support tel que défini précédemment, comprenant les étapes suivantes :
1) encapsulation d'au moins une substance active par (i) mise en contact de ladite au moins une substance active avec une solution d'encapsulation, (ii) mélange de ladite solution d'encapsulation dans un bain de gélification et (iii) extrusion du mélange sous forme de billes,
2) préparation de ladite matrice gélifiée par (i) mélange des composants de ladite matrice et d'un gélifiant, (ii) chauffage dudit mélange jusqu'à la température d'hydratation dudit gélifiant (iii) refroidissement du mélange pour être coulé dans un moule,
3) coulage du mélange obtenu à l'étape 2) dans ledit moule et introduction desdites billes dans ledit moule,
   ceci permettant l'obtention dudit support.

La première étape du procédé de l'invention permet donc l'encapsulation d'au moins une substance active sous forme de billes telles que définies ci-avant. Dans cette étape, la solution d'encapsulation peut être toute solution permettant la formation d'une enveloppe autour de la substance active. La solution d'encapsulation peut être par exemple composée d'alginates et/ou de cire végétale. La solution d'encapsulation peut comprendre en outre tout autre composé adapté, par exemple une ou plusieurs ingrédients choisis parmi les colorants, les agents de charge, comme les amidons. Le bain de gélification permet de gélifier la substance d'encapsulation et de lui donner la forme d'une enveloppe autour de la substance active. Le bain de gélification peut être composé de toute substance adaptée connue de l'homme du métier, comme par exemple du chlorure de calcium et/ou de l'acide citrique. Le bain de gélification comprenant la solution d'encapsulation et la substance active et ensuite soumis à une étape d'extrusion afin de donner au mélange la forme de billes. L'extrusion peut être réalisée par tout procédé adapté connu de l'homme du métier, comme par exemple par dripping dans le bain de gélification. Enfin les billes formées peuvent être filtrées puis rincées et séchées.

Dans le cas où l'on souhaite préparer un support contenant plusieurs sortes de substances actives, chaque bille contenant une substance active particulière est réalisée indépendamment des autres par la mise en oeuvre de l'étape 1) du procédé. En d'autres termes, les différentes substances actives ne sont pas mélangées dans l'étape 1), mais sont encapsulées indépendamment les unes des autres par la mise en oeuvre indépendante de l'étape 1). On réalise donc autant de fois l'étape 1) qu'il y a de substances actives différentes à encapsuler. Les étapes 1) peuvent être réalisées de manière de manière successive

La deuxième étape du procédé de l'invention est la préparation de la matrice telle que définie ci-avant. Les composants de la matrice sont tout d'abord mélangés avec au moins un gélifiant tel que décrit ci-avant. Puis, le mélange composé des composants de la matrice et du ou des gélifiant(s) est chauffé jusqu'à la température d'hydratation du gélifiant. Avantageusement, le mélange s'épaissit petit à petit en atteignant la température de gélification qui est inférieure à la température d'hydratation. Cette température d'hydratation, qui est la température T°c à laquelle s'activent les propriétés du gélifiant, est un paramètre physique connu de l'homme du métier, qui pourra la déterminer en fonction des composés présents dans le mélange. En général, la température d'hydratation est comprise entre 75 et 90°C, en fonction des gélifiants. Enfin, le mélange est refroidi jusqu'à ce que sa consistance soit adaptée à être coulée dans un moule. Avantageusement, l'acide citrique et/ou l'acide propionique et/ou l'acide butyrique et/ou l'acide isovalérique peu(ven)t permettre d'abaisser le pH.

Dans la première étape et dans la deuxième étape, l'Aw peut être contrôlée par ajout d'au moins un humectant, comme par exemple le monopropylene glycol ou le xilytol.

La troisième étape est l'étape de coulage du mélange refroidi dans un moule. Le moule peut avoir une forme adaptée pour conférer au support de l'invention la forme souhaitée. Une fois le mélange coulé dans le moule, les billes sont introduites, de préférence de manière homogène, dans le moule, c'est-à-dire dans le mélange constituant la matrice, ce qui permet d'obtenir un support comprenant une matrice dans laquelle sont incluses des billes contenant au moins une substance active. Afin de pouvoir incorporer facilement les billes au mélange, les billes y sont introduites avant que la matrice ne soit complètement gélifiée. Le support ayant la consistance souhaitée est ensuite obtenu en laissant le mélange dans lequel les billes sont incluses refroidir.

Un procédé non thérapeutique destiné à nourrir des animaux, en particulier des chiens et des chats **est décrit,** comprenant les opérations suivantes :
a) fabrication d'un support tel que défini ci-avant, et
b) administration dudit support auxdits animaux.
Dans ce cadre, le support ne contient pas de médicaments ni aucune substance active susceptible de traiter ou de prévenir une pathologie d'un animal.

D'autres avantages pourront encore apparaître à l'homme du métier à la lecture des exemples ci-dessous, illustrés par les figures annexées, donnés à titre illustratif.

### Brève description des figures

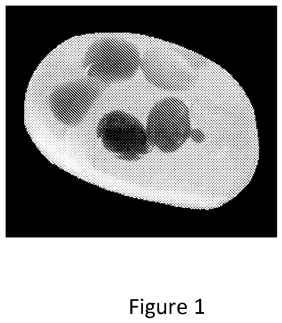

- La figure 1 représente une vue de dessus d'un support transparent de forme ovale comprenant 5 billes de différentes couleurs.

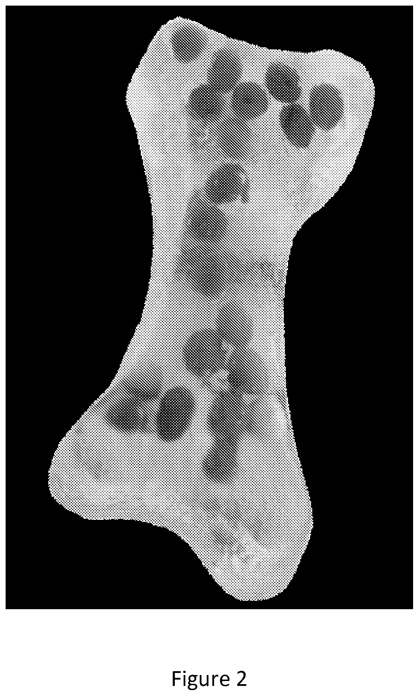

- La figure 2 représente une vue de dessus d'un support transparent de la forme d'un os comprenant 20 billes de différentes couleurs.

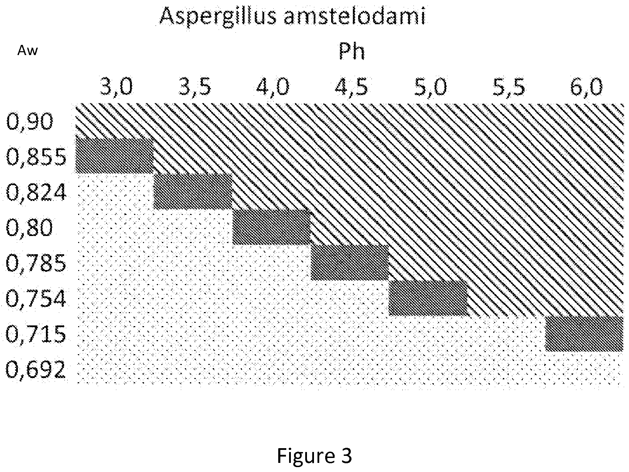

- La figure 3 représente le développement d'Aspergillus amstelodami dans un support en fonction du pH de la matrice (abscisse) et de l'Aw (ordonnée) de la matrice et des billes. Les hachures représentent une croissance d'Aspergillus amstelodami, les points représentent une absence de croissance d'Aspergillus amstelodami et les surfaces grises une croissance faible ou très ralentie d'Aspergillus amstelodami.

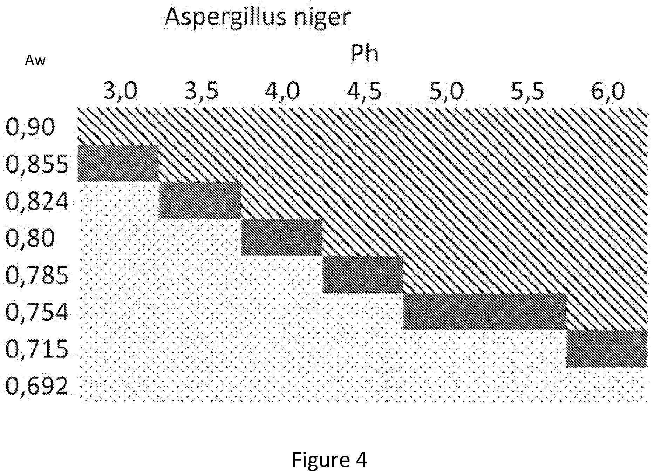

- La figure 4 représente le développement d'Aspergillus niger dans un support en fonction du pH (abscisse) et de l'Aw (ordonnée) de la matrice et des billes. Les hachures représentent une croissance d'Aspergillus niger, les points représentent une absence de croissance d'Aspergillus niger et les surfaces grises une croissance faible ou très ralentie d'Aspergillus niger.

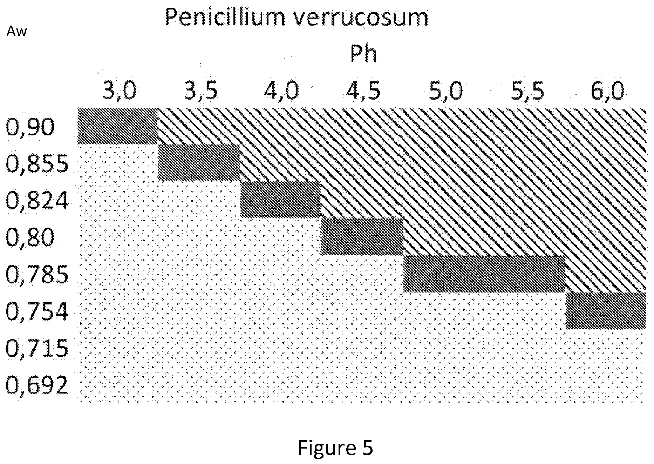

- La figure 5 représente le développement de Penicillium verrucosum dans un support en fonction du pH (abscisse) et de l'Aw (ordonnée) de la matrice et des billes. Les hachures représentent une croissance de Penicillium verrucosum, les points représentent une absence de croissance de Penicillium verrucosum et les surfaces grises une croissance faible ou très ralentie de Penicillium verrucosum.

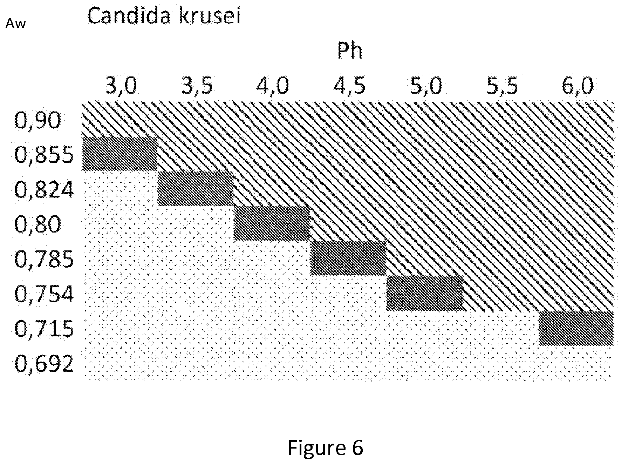

- La figure 6 représente le développement de Candida krusei dans un support en fonction du pH (abscisse) et de l'Aw (ordonnée) de la matrice et des billes. Les hachures représentent une croissance de Candida krusei, les points représentent une absence de croissance de Candida krusei et les surfaces grises une croissance faible ou très ralentie de Candida krusei.

### EXEMPLES

### Exemple 1 : fabrication de friandises gélifiées

La première étape du procédé de fabrication consiste en l'encapsulation de matières actives sous forme de billes d'alginates ou de cire végétale. Ces billes comprennent entre 20 et 70% de matières actives et sont d'un diamètre compris entre 200 microns à 6 mm. Leur AW (eau résiduelle) est comprise entre 0,4 et 0,9.

La fabrication des billes consiste en la préparation d'une solution d'encapsulation composée d'alginates ou de cire végétale.

Le bain de gélification est ensuite préparé. Celui-ci est constitué de CaCl₂ et d'acide citrique.

Les actifs ainsi que les agents de charges (amidons) et les colorants sont incorporés dans la solution d'encapsulation. Puis le mélange est extrudé par dripping dans le bain de gélification.

Enfin les billes formées sont filtrées puis rincées et séchées sur un lit d'air fluidisé (70°c).

La deuxième étape consiste en la préparation de 2 mélanges.

Un 1^{er} mélange comprenant toutes les matières premières liquides de la composition est réalisé. Celui-ci est composé de sirop de glucose, de glycérol, d'eau, de facteur d'appétence et d'huile (végétale ou huile de poisson). Ce mélange est chauffé jusqu'à dissolution complète des matières premières plus consistantes.

Un 2^{nd} mélange comprenant les matières premières sous forme de poudre est préparé. Celui-ci contient le gélifiant (carraghénanes, agar agar, gomme gellan, gélatine de boeuf, porc ou poisson, gomme xanthane, farine de graine de caroube, gomme de guar, gomme arabique, alginates, gomme adragante tragacanthe ou amidons), la solution appétente, le sorbate de potassium, le citrate de sodium, le citrate tri potassique, le sel, le dextrose.

Les différents ingrédients liquides (eau, sirop de glucose, glycérol) de la matrice sont mis en solution, et chauffés jusqu'à dissolution complète des matières premières liquides. Lorsque que l'ensemble des liquides est fondu, on introduit le noyau des matières premières en poudres.

Le mélange est ensuite porté jusqu'à la température d'hydratation du gélifiant utilisé (entre 75 et 90°C).

Puis on laisse redescendre la température du mélange à 70°C et introduit l'acide citrique.

On laisse refroidir le mélange et on attend qu'il prenne un peu en viscosité.

Le mélange est ensuite coulé dans les blisters et les billes contenant les différentes matières actives sont introduites dans chaque GELICAPS, et réparties de manière homogène.

### Exemple 2 : Exemple de composition d'un support

Un support de l'invention, présentée comme une friandise pour chien, est préparée par la mise en oeuvre du procédé décrit à l'étape 1, et possède la composition suivante :
Billes :

| | **POURCENTAGE EN POIDS DANS LA BILLE** |
|---|---|
| Alginates | 0,1 à 5% |
| Amidons | 2 à 50% |
| Colorants | 0.05 à 2% |
| Substance active | 10 à 90% |

Matrice :

| | |
|---|---|
| Eau | 30.775% |
| Sirop de glucose | 20% |
| Glycérol | 18% |
| Facteur d'appétance | 5% |
| Huile de poisson | En enrobage 1% ou dans la matrice |
| Gélatine de boeuf | 12.5% |
| Sorbate de potassium | 0.4% |
| Citrate de sodium | 0.1% |
| Citrate tripotassique | 0.075% |
| Sel | 2% |
| Dextrose | 9.55% |

### Exemple 3 : Evolution de la stabilité du support en fonction du pH

Des supports ont été préparés en suivant le protocole décrit à l'exemple 1, tout en contrôlant le pH et l'Aw de la matrice et des billes de chacun de ces supports. Pour tous les supports, les valeurs de l'Aw sont identiques dans les billes et dans le support. Les pH obtenus ont les valeurs suivantes : 3,0, 3,5, 4,0, 4,5, 5,0, 5,5, 6,0.

L'Aw est contrôlée par contrôle de la quantité de glycérol et/ou xylitol. Pour chaque valeur de pH de la matrice, 8 supports sont réalisé dans lesquels les ingrédients liquides de la matrices et l'Aw sont décrits dans le tableau suivant :

| Glycérol (grammes) | Xylitol (grammes) | Eau (grammes) | Aw |
|---|---|---|---|
| 500 | | 1000 | 0,90 |
| 750 | | 1000 | 0,855 |
| 900 | | 1000 | 0,824 |
| 1350 | | 1000 | 0,80 |
| 1350 | 900 | 1000 | 0,715 |
| 1100 | 600 | 1000 | 0,785 |
| 1100 | 900 | 1000 | 0,754 |
| 1500 | 900 | 1000 | 0,692 |

La stabilité de chaque support est ensuite testée, en ensemençant chaque support par les souches suivantes : Aspergillus amstelodami, Aspergillus niger, Pénicillium verrucosum et Candida krusei.

Les cultures ont été réalisées à 25°C, dans l'obscurité, pendant 15 jours. Concernant les moisissures, l'ensemencement est réalisé par la périphérie d'un thalle en croissance (les tests ont porté sur la mesure de la croissance des thalles) ; les levures ont été ensemencées en goutte (contenant environ 10^6 CFU/ml) soit environ 10^3 CFU.

Les résultats sont décrits respectivement dans les figures 3, 4, 5 et 6. Ils montrent une stabilité du support à un pH inférieur ou égal à 4,0, pour tous les microorganismes testés.

## Revendications

1. Support permettant de faciliter l'absorption orale d'au moins une substance active par des animaux, **caractérisé en ce qu'**il comprend :
- au moins une bille comprenant ladite au moins une substance active,
- une matrice appétente gélifiée entourant ladite au moins une bille, le pH de ladite matrice étant inférieur à 4,0,
dans lequel l'Aw (eau résiduelle) de ladite matrice et de ladite au moins une bille est identique et comprise de 0,4 à 0,9.

2. Support selon la revendication 1, dans lequel ladite bille est une capsule ou une microcapsule.

3. Support selon la revendication 2, dans lequel ladite capsule ou microcapsule est une capsule ou une microcapsule d'alginate ou de cire végétale.

4. Support selon l'une quelconque des revendications précédentes, comprenant au moins 2 billes, chacune des billes comprenant une substance active pouvant être identique et/ou différente de celles de l'autre ou des autres billes.

5. Support selon l'une quelconque des revendications précédentes, dans laquelle ladite matrice comprend un gélifiant choisi parmi les carraghénanes, l'agar agar, la gomme gellane, la gélatine de boeuf, de porc ou de poisson, la gomme xanthane, la farine de graine de caroube, la gomme de guar, la gomme arabique, les alginates, la gomme adragante et les amidons.

6. Support selon l'une quelconque des revendications précédentes, dans lequel ladite au moins une substance active est choisie dans le groupe comprenant les probiotiques, les huiles essentielles, les médicaments, les nutraceutiques et les nutriments.

7. Support selon l'une quelconque des revendications précédentes, dans lequel ladite matrice comprend en outre de la cellulose microcristalline.

8. Support tel que défini dans l'une quelconque des revendications 1 à 5, pour son utilisation comme médicament destiné aux animaux, dans lequel ladite au moins une substance active comprise dans l'au moins une bille est au moins un médicament.

9. Procédé de préparation d'un support tel que défini dans l'une quelconque des revendications 1 à 7, comprenant les étapes suivantes :
1) encapsulation d'au moins une substance active par (i) mise en contact de ladite au moins une substance active avec une solution d'encapsulation, (ii) mélange de ladite solution d'encapsulation dans un bain de gélification et (iii) extrusion du mélange sous forme de billes,
2) préparation d'une matrice gélifiée par (i) mélange des composants de ladite matrice et d'un gélifiant, (ii) chauffage dudit mélange jusqu'à la température d'hydratation dudit gélifiant (iii) refroidissement du mélange pour être coulé dans un moule,
3) coulage du mélange obtenu à l'étape 2) dans ledit moule et introduction desdites billes dans ledit moule,
ceci permettant l'obtention dudit support.

## Patentansprüche

1. Träger, der es gestattet, die orale Aufnahme mindestens eines Wirkstoffs durch Tiere zu erleichtern, **dadurch gekennzeichnet, dass** er Folgendes umfasst:
- mindestens ein Kügelchen, das den mindestens einen Wirkstoff umfasst,
- eine schmackhafte Gelmatrix, die das mindestens eine Kügelchen umgibt, wobei der pH-Wert der Matrix weniger als 4,0 beträgt,
wobei das Aw (Restwasser) der Matrix und des mindestens einen Kügelchens identisch ist und zwischen 0,4 und 0,9 beträgt.

2. Träger nach Anspruch 1, wobei das Kügelchen eine Kapsel oder eine Mikrokapsel ist.

3. Träger nach Anspruch 2, wobei die Kapsel oder Mikrokapsel eine Kapsel oder Mikrokapsel aus Alginat oder pflanzlichem Wachs ist.

4. Träger nach einem der vorhergehenden Ansprüche, umfassend mindestens 2 Kügelchen, wobei jedes der Kügelchen einen Wirkstoff umfasst, der mit denjenigen des anderen oder der anderen Kügelchen identisch und/oder verschieden davon sein kann.

5. Träger nach einem der vorhergehenden Ansprüche, wobei die Matrix ein Geliermittel umfasst, das aus Carrageenen, Agar-Agar, Gellangummi, Rinder-, Schweine- oder Fischgelatine, Xanthangummi, Johannisbrotkernmehl, Guarkernmehl, Gummi arabicum, Alginaten, Tragantgummi und Stärken ausgewählt ist.

6. Träger nach einem der vorhergehenden Ansprüche, wobei der mindestens eine Wirkstoff aus der aus Probiotika, ätherischen Ölen, Arzneimitteln, Nutrazeutika und Nährstoffen bestehenden Gruppe ausgewählt ist.

7. Träger nach einem der vorhergehenden Ansprüche, wobei die Matrix außerdem mikrokristalline Cellulose umfasst.

8. Träger nach einem der Ansprüche 1 bis 5 zur Verwendung als Arzneimittel für Tiere, wobei der mindestens eine in dem mindestens einen Kügelchen enthaltene Wirkstoff mindestens ein Arzneimittel ist.

9. Verfahren zur Herstellung eines Trägers nach einem der Ansprüche 1 bis 7, umfassend die folgenden Schritte:
1) Einkapselung mindestens eines Wirkstoffs durch (i) Inkontaktbringen des mindestens einen Wirkstoffs mit einer Einkapselungslösung, (ii) Einmischen der Einkapselungslösung in ein Gelierungsbad und (iii) Extrusion des Gemischs in Form von Kügelchen,
2) Herstellen einer Gelmatrix durch (i) Mischen der Bestandteile der Matrix und eines Geliermittels, (ii) Erhitzen des Gemischs auf die Hydratationstemperatur des Geliermittels, (iii) Abkühlen des Gemischs für das Gießen in eine Form,
3) Gießen des in Schritt 2) erhaltenen Gemischs in die Form und Einbringen der Kügelchen in die Form, wodurch sich der Träger erhalten lässt.

## Claims

1. Support for facilitating the oral absorption of at least one active substance by animals, **characterized in that** it comprises:
- at least one bead comprising said at least one active substance,
- a gelled appetizing matrix surrounding said at least one bead, the pH of said matrix being less than 4.0,
in which the Aw (residual water) of said matrix and of said at least one bead is identical and between 0.4 and 0.9.

2. Support according to Claim 1, in which said bead is a capsule or a microcapsule.

3. Support according to Claim 2, in which said capsule or microcapsule is an alginate or plant wax capsule or microcapsule.

4. Support according to any one of the preceding claims, comprising at least two beads, each of the beads comprising an active substance which may be identical to or different from those of the other bead(s).

5. Support according to any one of the preceding claims, in which said matrix comprises a gelling agent chosen from carrageenans, agar agar, gellan gum, beef, pig or fish gelatin, xanthan gum, locust bean meal, guar gum, acacia gum, alginates, gum tragacanth and starches.

6. Support according to any one of the preceding claims, in which said at least one active substance is chosen from the group comprising probiotics, essential oils, medicaments, neutraceuticals and nutrients.

7. Support according to any one of the preceding claims, in which said matrix also comprises microcrystalline cellulose.

8. Support as defined in any one of Claims 1 to 5, for its use as a medicament intended for animals, in which said at least one active substance included in the at least one bead is at least one medicament.

9. Process for preparing a support as defined in any one of Claims 1 to 7, comprising the following steps:
1) encapsulation of at least one active substance by (i) placing said at least one active substance in contact with an encapsulating solution, (ii) mixing said encapsulating solution in a gelling bath and (iii) extruding the mixture in the form of beads,
2) preparation of a gelled matrix by (i) mixing the components of said matrix and a gelling agent, (ii) heating said mixture up to the hydration temperature of said gelling agent and (iii) cooling the mixture to pour it into a mould,
3) pouring of the mixture obtained in step 2) into said mould and introduction of said beads into said mould,
thus enabling said support to be produced.
